# EUROPEAN PATENT SPECIFICATION

(11) **EP 3 718 474 B1**
(45) Date of publication and mention of the grant of the patent: **15.02.2023**
(21) Application number: 18898068.4
(22) Date of filing: 31.12.2018
(51) Int. Cl.: A61B 5/055, G01R 33/56, G01R 33/483

(54) **METHOD FOR PROVIDING GUIDE INFORMATION ASSOCIATED WITH ARTIFACTS AND MAGNETIC RESONANCE IMAGING DEVICE THEREFOR**
VERFAHREN ZUR BEREITSTELLUNG VON FÜHRUNGSINFORMATIONEN IM ZUSAMMENHANG MIT ARTEFAKTEN UND MAGNETRESONANZBILDGEBUNGSVORRICHTUNG DAFÜR
PROCÉDÉ DE FOURNITURE D'INFORMATIONS DE GUIDAGE ASSOCIÉES À DES ARTEFACTS ET DISPOSITIF D'IMAGERIE PAR RÉSONANCE MAGNÉTIQUE ASSOCIÉ

(30) Priority: 02.01.2018 KR 20180000267
(43) Date of publication of application: 07.10.2020
(73) Proprietor: Samsung Electronics Co., Ltd., Gyeonggi-do 16677 (KR)
(72) Inventor: LEE, Daeho, Suwon-si, Gyeonggi-do 16677 (KR); CHOI, Joonsung, Suwon-si, Gyeonggi-do 16677 (KR)
(74) Representative: Gulde & Partner
(86) International application number: PCT/KR2018/016967
(87) International publication number: WO 2019/135578

(56) References cited:
- EP-A2- 1 004 891
- JP-A- 2003 000 564
- JP-A- 2007 260 292
- JP-A- 2008 125 814
- KR-A- 20140 148 242
- KR-B1- 101 438 758
- US-A1- 2010 201 360
- US-A1- 2010 253 335
- US-A1- 2016 033 600
- US-A1- 2016 231 409
- US-A1- 2017 343 635

## Description

### TECHNICAL FIELD

The present disclosure relates to a method and apparatus for predicting the occurrence of artifacts and preventing the artifacts.

### BACKGROUND ART

A magnetic resonance imaging (MRI) system obtains a magnetic resonance (MR) signal and reconstructs the obtained MR signal as an image. The MR signal refers to a radio frequency (RF) signal radiated from an object.

An MRI system forms a static magnetic field and aligns magnetic dipole moments of specific atomic nuclei of an object located in the static magnetic field in a direction of the static magnetic field. A gradient magnetic field coil may form a gradient magnetic field by applying a gradient signal to a static magnetic field and induce a resonance frequency differently for each part of an object.

An RF coil may emit an RF signal according to a resonance frequency of a region to be imaged. Furthermore, as the gradient magnetic field is formed, the RF coil may receive MR signals of different resonance frequencies radiated from various parts of the object. Through these operations, the MRI system obtains an image from an MR signal by using an image reconstruction technique.

However, artifacts may be generated in the generated MR image. Therefore, there is a need for technology for predicting the occurrence of artifacts before MR imaging and preventing the artifacts.

US 2016/033600 A1 and US 2010/201360 A1 are concerned with MRI system that allow for artefacts in the MR image to be reduced. US-2010/253335-A1, US-2017/343635-A1 and US-2016/231409-A1 show further artefact reducing techniques. EP-1004891-A2 shows a user interface for MRI systems.

### DESCRIPTION OF EMBODIMENTS

### TECHNICAL PROBLEM

In order to obtain a magnetic resonance (MR) image, there is a need for technology for predicting the occurrence of artifacts based on a signal region and a region of interest (ROI) and preventing the artifacts.

### SOLUTION TO PROBLEM

The invention is defined by the claims. According to an aspect of the present disclosure, a magnetic resonance imaging (MRI) device includes: a display configured to display a first image of an object; a controller configured to determine a signal region of the object based on the first image of the object; and a user input device configured to receive a user input of setting a region of interest (ROI) on the first image, wherein, when the set ROI is part of the signal region of the object, the controller is further configured to control the display to display guide information for preventing aliasing artifacts from occurring in a second image to be generated based on the set ROI.

### ADVANTAGEOUS EFFECTS OF DISCLOSURE

More efficient and precise magnetic resonance (MR) images may be obtained by predicting the occurrence of artifacts before MR imaging and preventing the artifacts.

### BRIEF DESCRIPTION OF DRAWINGS

FIG. 1 illustrates a method, performed by a magnetic resonance imaging (MRI) device, of providing guide information for preventing artifacts, according to some embodiments.
FIG. 2 is a flowchart of a method, performed by an MRI device, of providing guide information for preventing artifacts, according to some embodiments.
FIG. 3 is a diagram illustrating a method of determining whether aliasing will occur when a user input of setting a region of interest (ROI) on a first magnetic resonance (MR) image is received, according to some embodiments.
FIG. 4 illustrates a method, performed by an MRI device, of providing a user interface (Ul) for adjusting a length of an ROI so as to prevent artifacts, according to some embodiments.
FIG. 5 illustrates a method, performed by an MRI device, of providing an outer-volume suppression (OVS) method of limiting a signal of a region where aliasing occurs, so as to prevent artifacts, according to some embodiments.
FIGS. 6 and 7 illustrate a method, performed by an MRI device, of setting a multi-band, according to some embodiments.
FIG. 8 illustrates a method, performed by an MRI device, of providing guide information to allow an MR signal to be generated only from an ROI among pieces of guide information for preventing artifacts, according to some embodiments.
FIG. 9 is a schematic diagram of an MRI device.

### BEST MODE

According to an aspect of the present disclosure, a magnetic resonance imaging (MRI) device includes: a display configured to display a first image of an object; a controller configured to determine a signal region of the object based on the first image of the object; and a user input device configured to receive a user input of setting a region of interest (ROI) on the first image, wherein, when the set ROI is part of the signal region of the object, the controller is further configured to control the display to display guide information for preventing aliasing artifacts from occurring in a second image to be generated based on the set ROI.

Furthermore, the controller may be further configured to determine a predicted image based on the signal region and the ROI, and the display may be further configured to display the predicted image.

Furthermore, the display may be further configured to display a position of a region where aliasing occurs in the signal region of the first image.

Furthermore, the controller may be further configured to display the guide information by displaying information about a method of increasing a length of the ROI in a phase direction so that the ROI includes a signal region in the phase direction.

Furthermore, the controller may be further configured to display the guide information by displaying information about a method of suppressing a magnetic resonance (MR) signal emitted from a region other than the ROI in the signal region.

Furthermore, the controller may be further configured to display the guide information by displaying information about a method of emitting an MR signal corresponding to an imaging sequence of the second image from the ROI of the signal region.

Furthermore, the method of suppressing the MR signal may include determining a pair of regions parallel to each other in a region surrounding the ROI, and setting a multi-saturation band for simultaneously suppressing MR signals emitted from the determined pair of regions.

According to the invention the guide information includes a plurality of icons corresponding to a plurality of methods of preventing the aliasing artifacts from occurring, and when a user input of selecting one of the plurality of icons is received, the controller is further configured to display guide information about a method corresponding to the selected icon.

Furthermore, the controller may be further configured to display an estimated imaging time of each of the plurality of methods together with the plurality of icons corresponding to the plurality of methods.

Furthermore, the controller may be further configured to determine the guide information based on coil geometry of a receiving coil, a shape of the ROI, anatomical information about the ROI, and a pulse sequence.

### MODE OF DISCLOSURE

The present specification describes principles of the present disclosure and sets forth embodiments thereof to clarify the scope of the present disclosure and to allow those of ordinary skill in the art to implement the embodiments. The present embodiments may have different forms.

Like reference numerals refer to like elements throughout. The present specification does not describe all components in the embodiments, and common knowledge in the art or the same descriptions of the embodiments will be omitted below. The term "part" or "portion" may be implemented using hardware or software, and according to embodiments, one "part" or "portion" may be formed as a single unit or element or include a plurality of units or elements. Hereinafter, the principles and embodiments of the present disclosure will be described in detail with reference to the accompanying drawings.

In the present specification, an "image" may include a medical image obtained by a magnetic resonance imaging (MRI) device.

Furthermore, in the present specification, an "object" may be a target to be imaged and include a human, an animal, or a part of a human or animal. For example, the object may include a body part (an organ) or a phantom.

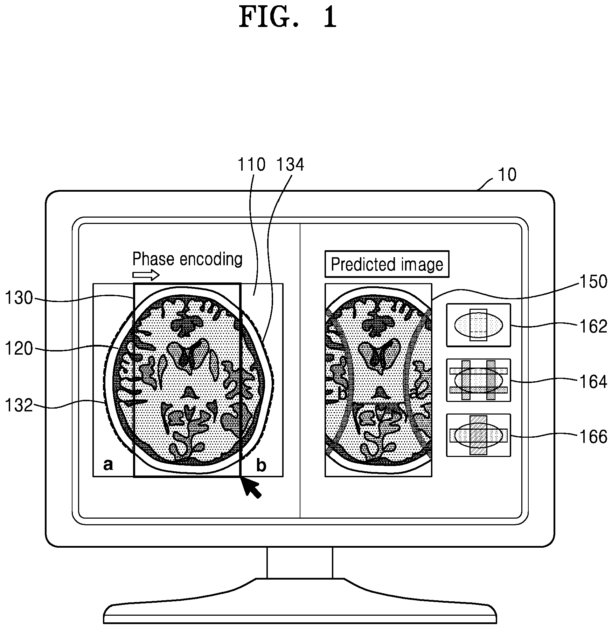

FIG. 1 illustrates a method, performed by an MRI device 1, of providing guide information for preventing artifacts, according to some embodiments.

A device illustrated in FIG. 1 represents an operating unit 10 of the MRI device 1.

Referring to FIG. 1, the MRI device 1 may display a first magnetic resonance (MR) image 110 for setting a region of interest (ROI) 130. Furthermore, the MRI device 1 may receive a user input of setting the ROI 130 on the first MR image 110. When the MRI device 1 receives the user input of setting the ROI 130 on the first MR image 110, before the MRI device 1 generates a second MR image for the determined ROI 130, the MRI device 1 may determine whether aliasing will occur when the second MR image for the ROI 130 is generated.

When the MRI device 1 expects that aliasing will occur when the second MR image is generated, the MRI device 1 may provide guide information for preventing aliasing.

For example, the MRI device 1 may provide a method (ROI resizing method) of increasing the ROI in a phase encoding direction. Furthermore, the MRI device 1 may provide a method (outer-volume suppression (OVS) method) of setting a region other than the ROI in a signal region as a saturation region. Furthermore, the MRI device 1 may provide a method (inner-volume imaging (IVI) method) of allowing an MR signal to be emitted only from the ROI.

Furthermore, when the MRI device 1 determines that aliasing will occur when the second MR image is generated, the MRI device 1 may generate a predicted image 150 based on the determined ROI and display the generated predicted image 150.

When a brain of an object is imaged, the first MR image 110 may be a brain image of the object. The first MR image 110 may be a plane image obtained by roughly imaging the brain of the object so as to set a region to be imaged or may be the brain image of the object that is previously imaged and stored in the MRI device 1. A user may set the ROI on the first MR image 110.

The MRI device 1 may determine a signal region in the first MR image 110 based on the first MR image 110. The signal region may refer to a region in which the MR signal is received by the MRI device 1 in the region of the object. In the case of the first MR image 110 illustrated in FIG. 1, the MRI device 1 may determine the inside of a brain boundary in the first MR image 110 as the signal region 120.

When the determined ROI 130 does not include the entire signal region 120 in the phase encoding direction, the MRI device 1 may determine that aliasing will occur.

When the MRI device 1 determines that aliasing will occur, the MRI device 1 may generate the predicted image 150. For example, since an "a" region 132 and a "b" region 134 in the page encoding direction that are not included in the ROI 130 in the signal region 120 of the first MR image 110 are folded over in an opposite direction within the ROI 130, the MRI device 1 may generate the predicted image 150 by determining an image indicating the ROI 130 based on the first MR image, displaying the "a" region 132, located on the left side of the ROI, on the right side of the determined image in a superimposed manner, and displaying the "b" region 134, located on the right side of the ROI, on the left side of the determined image in a superimposed manner.

The MRI device 1 may display the generated predicted image 150 so as to provide, to the user, information indicating that artifacts will be included in the second MR image.

Furthermore, when the MRI device 1 determines that aliasing will occur, the MRI device 1 may provide a UI for performing a method of preventing aliasing. For example, the MRI device 1 may provide an icon 162 for performing an ROI resizing method, an icon 164 for performing an OVS method, and an icon 166 for performing an IVI method. Each icon may include an image or a text indicating each method.

Furthermore, the MRI device 1 may calculate an estimated imaging time for each method and display the calculated estimated imaging time together with the icon indicating each method.

The first MR image 110 and the second MR image may each be a two-dimensional (2D) image or a three-dimensional (3D) image.

Throughout the specification, each method has been described based on the ROI, but when the MRI device 1 determines the ROI as a field of view (FOV), the ROI may be replaced with the FOV.

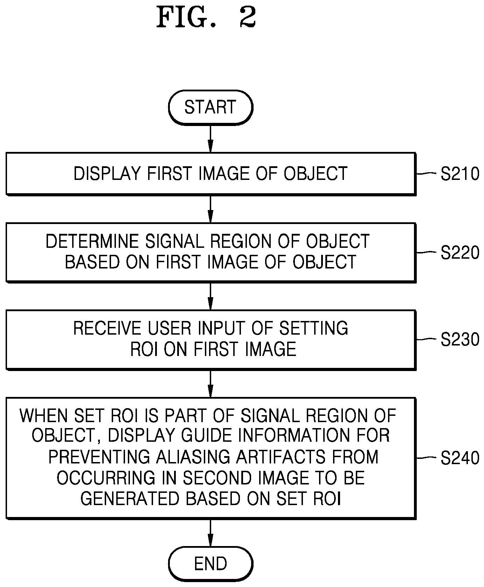

FIG. 2 is a flowchart of a method, performed by the MRI device 1, of providing guide information for preventing artifacts, according to some embodiments.

In operation S210, the MRI device 1 may display a first image of an object.

When capturing an MR image, it is a general imaging process to capture an approximate image of an object so as to set an accurate ROI before imaging the ROI. For example, when capturing a cardiac image of an object, the user may obtain an approximate first image by imaging a body of the object and set a cardiac region in the first image as an ROI.

The first MR image may be a plane image obtained by roughly imaging an object so as to set a region to be imaged or may be an image of an object that is previously imaged and stored in the MRI device 1.

In operation S220, the MRI device 1 may determine a signal region of the object based on the first image of the object.

The signal region may refer to a region in which the MR signal is received by the MRI device 1 in the region of the object. The MRI device 1 may identify a region of the first image in which the object is displayed and determine the identified region as the signal region. In the case of the first MR image illustrated in FIG. 1, the MRI device 1 may determine the inside of the brain boundary in the first MR image as the signal region.

In operation S230, the MRI device 1 may receive a user input of setting an ROI on the first image.

In operation S240, when the set ROI is part of the signal region of the object, the MRI device 1 may display guide information for preventing aliasing artifacts from occurring in a second image generated based on the set ROI.

When the set ROI is part of the signal region of the object, aliasing may occur when capturing the MR image due to the MR signal received from the region outside the ROI in the signal region. Therefore, aliasing artifacts may be included in the second image generated based on the ROI.

The MRI device 1 may provide various methods of preventing aliasing artifacts from being included in the second image generated based on the ROI.

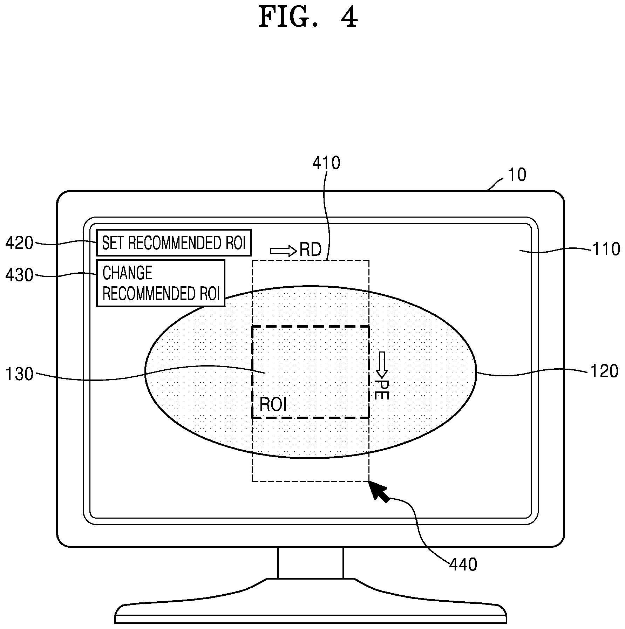

For example, the MRI device 1 may provide information about a method of increasing the length of the ROI in the phase direction so that the ROI includes the signal region in the phase direction. This embodiment will be described in detail below with reference to FIG. 4.

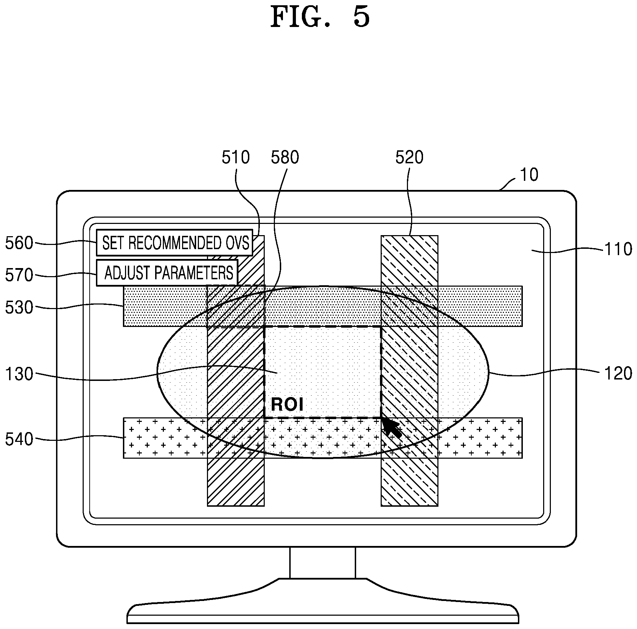

In addition, for example, the MRI device 1 may provide information about an OVS method of suppressing an MR signal emitted from a region other than an ROI in a signal region. This embodiment will be described in detail below with reference to FIGS. 5 to 7.

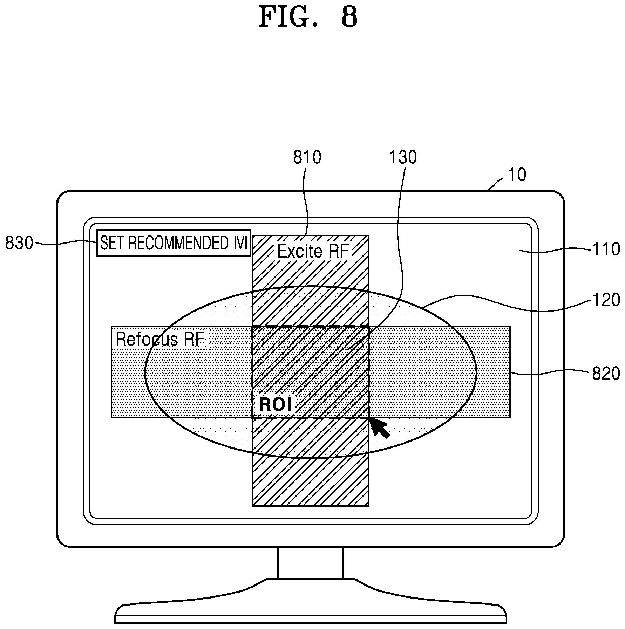

Furthermore, for example, the MRI device 1 may provide an IVI method of emitting an MR signal corresponding to an imaging sequence of a second image only from an ROI in a signal region. This embodiment will be described in detail below with reference to FIG. 8.

The MRI device 1 may recommend a method of simultaneously applying two or more of a plurality of methods. For example, the MRI device 1 may recommend a method of simultaneously applying the IVI method and the OVS method.

The MRI device 1 may determine guide information based on coil geometry of a receiving coil, a shape of an ROI, anatomical information about an ROI, and a pulse sequence. For example, the MRI device 1 may determine a saturation region when applying the OVS method, based on the coil geometry of the receiving coil. Furthermore, the MRI device 1 may determine whether the IVI method is applicable, based on the shape of the ROI, and may determine the number of saturation bands and the area of the band when applying the OVS method. Furthermore, the MRI device 1 may determine the ROI based on the anatomical information about the ROI and determine a method most suitable for the ROI. Furthermore, the MRI device 1 may determine whether the IVI method is applicable, based on the pulse sequence.

The MRI device 1 may display guide information for reducing a scan time separately from artifacts. For example, when the long side of the ROI is set as a phase encoding direction and the short side of the ROI is set as a read-out direction, the scan time is reduced when the length in the phase encoding direction is short. Therefore, guide information may be provided so that the long side of the ROI is set as the read-out direction and the short side of the ROI is changed to the phase encoding direction.

The MRI device 1 may calculate a scan time expected to be required when generating a second image according to each method and may display the calculated scan time.

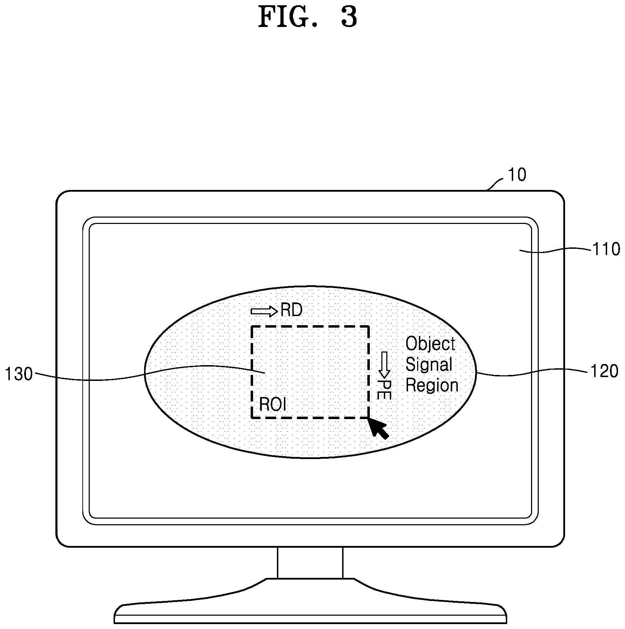

FIG. 3 is a diagram illustrating a method of determining whether aliasing will occur when a user input of setting an ROI on a first MR image is received, according to some embodiments.

The MRI device 1 may receive a user input of setting an ROI on a first MR image 110. Furthermore, the MRI device 1 may receive a user input of setting a phase encoding direction and a read-out direction on the ROI.

In this case, as the length of the ROI in the phase encoding direction is smaller, the scan time may be further reduced. Therefore, the user may set the length of the ROI in the phase encoding direction as small as possible so as to reduce the scan time. However, as illustrated in FIG. 3, aliasing may occur when the ROI in the phase encoding direction does not cover a signal region. An inexperienced user may set the ROI so that the ROI in the phase encoding direction does not cover the signal region.

When the MRI device 1 receives the user input of setting the ROI, the MRI device 1 may determine whether aliasing will occur when a second image corresponding to the ROI is generated.

The MRI device 1 may compare the set ROI with the determined signal region. When the set ROI does not cover the signal region in the phase encoding direction, the MRI device 1 may determine that aliasing will occur when the second MR image is generated.

Furthermore, the MRI device 1 may determine a region expected to generate artifacts in the second MR image of the signal region, based on the position of the signal region and the position of the set ROI. For example, the MRI device 1 may determine a region in the phase encoding direction in the signal region, excluding the ROI, as the region expected to generate artifacts. Referring back to FIG. 1, the MRI device 1 may determine the "a" region 132 and the "b" region 134 of the signal regions as the region expected to generate artifacts. Furthermore, the MRI device 1 may display the region predicted to generate artifacts in the signal region as distinguished from other regions.

Referring back to FIG. 3, the read-out direction of the ROI also does not cover the signal region, but the MRI device 1 may automatically prevent aliasing by performing filtering or oversampling on the signal region in the read-out direction that is not covered by the ROI.

FIG. 4 illustrates a method, performed by the MRI device 1, of providing a UI for adjusting a length of an ROI so as to prevent artifacts, according to some embodiments.

Referring to FIG. 4, the MRI device 1 may display a recommended ROI 410 by increasing a length of an ROI 130 in a phase encoding direction so that the region of the ROI 130 in the phase encoding direction includes a signal region 120.

in this case, since a scan time increases as the length of the ROI increases in the phase encoding direction, the MRI device 1 may increase the length of the phase encoding direction so that the region in the phase encoding direction includes the signal region 120 and is located as close as possible to the signal region 120.

Furthermore, the MRI device 1 may display a button 420 for changing the ROI 130 to the recommended ROI 410. When the MRI device 1 receives a user input of clicking the button 420, the MRI device 1 may change the ROI 130 to the recommended ROI 410.

Furthermore, the MRI device 1 may display a button 430 for changing the recommended ROI 410. When the MRI device 1 receives a user input of clicking the button 430, the MRI device 1 may display a cursor for changing the recommended ROI 410. When the MRI device 1 receives a user input of moving a cursor 440, the MRI device 1 may reset the ROI based on the position of the cursor 440.

FIG. 5 illustrates a method, performed by the MRI device 1, of providing an OVS method of limiting a signal of a region where aliasing occurs so as to prevent artifacts, according to some embodiments.

Referring to FIG. 5, the MRI device 1 may limit the signal of the region where aliasing occurs by suppressing an MR signal emitted from a region outside an ROI.

When the MR signal emitted from the region outside the ROI is received as the signal of the ROI, artifacts may occur on a generated MR image. Therefore, the MRI device 1 may prevent aliasing artifacts by suppressing the MR signal emitted from the region outside the ROI before receiving an MR signal emitted from an object.

For example, the MRI device 1 may limit the signal of the region where aliasing occurs by generating a saturation pulse before a slice excitation pulse of an imaging sequence. Before acquiring a line of k-space, when the MR signal to be emitted from the region outside the ROI is suppressed by generating the saturation pulse for the region outside the ROI and then the MR signal is received from the object, the MR signal to be emitted from the region outside the ROI is suppressed. Therefore, since the MR signal to be emitted from the region outside the ROI does not enter the ROI, aliasing may not occur.

When the ROI is set, the MRI device 1 may display the saturation region by displaying saturation bands 510, 520, 530, and 540 in the region outside the ROI.

In this case, the saturation bands are set to surround the ROI. However, when the ROI is not a circular shape or a standardized shape, it is difficult for the user to directly set the saturation bands with respect to all regions surrounding the ROI. The MRI device 1 analyzes the shape of the ROI and the saturation bands surround the ROI, but the area, number, and positions of the saturation bands may be determined so that a space between the saturation bands and the ROI becomes minimum.

For example, as illustrated in FIG. 5, for the rectangular ROI, the MRI device 1 may determine the saturation region so that four saturation bands come into contact with four sides of the ROI.

In addition, the MRI device 1 may display a button 560 for setting a recommended saturation band. When the MRI device 1 receives a user input of selecting the button 560 for setting the recommended saturation band, the MRI device 1 may perform MR imaging based on the displayed saturation bands.

Furthermore, the MRI device 1 may display a button 570 for adjusting parameters of the recommended saturation band. When the MRI device 1 receives a user input of selecting the button 570 for adjusting the parameters, the MRI device 1 may provide a UI for centroid shift, rotation, or thickness adjustment of the saturation bands.

Furthermore, when the saturation bands are set, an MR signal may be generated in a region 580 in which the saturation bands cross each other. The MRI device 1 may prevent the performance of saturation from deteriorating in the region in which the saturation bands superimpose each other by applying a phase off-set between RF pulses corresponding to the saturation bands crossing each other.

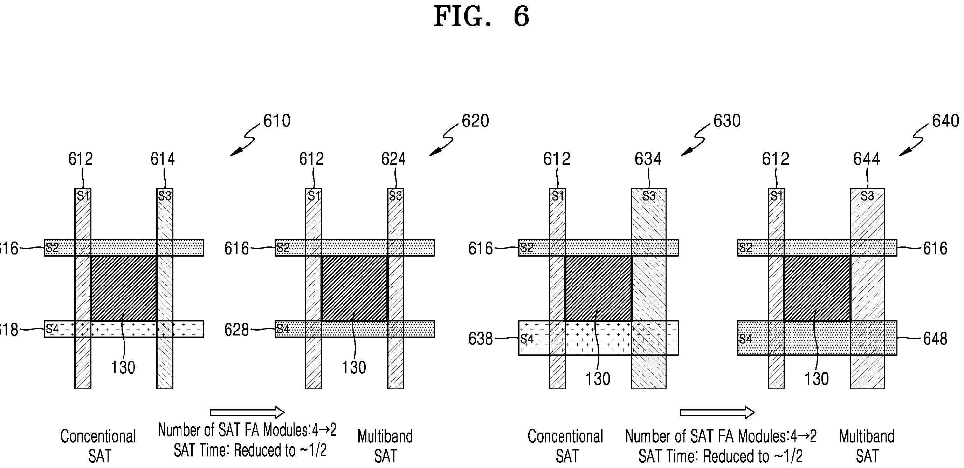

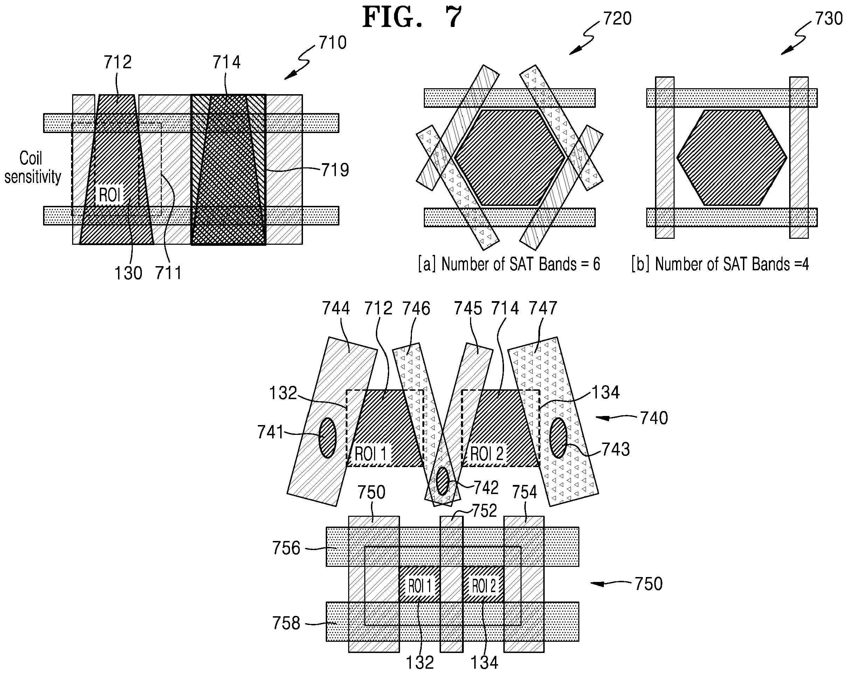

FIGS. 6 and 7 illustrate a method, performed by the MRI device 1, of setting a multi-band, according to some embodiments.

Referring to FIGS. 6 and 7, the MRI device 1 may recommend the multi-band that simultaneously generates corresponding pulses with respect to parallel saturation bands among saturation bands.

By applying the multi-band during MR imaging, the MRI device 1 may reduce the number of RF modules for saturating an MR signal and may reduce the time taken to saturate the MR signal.

For example, reference numeral 610 of FIG. 6 shows that the MRI device 1 sets general saturation bands, according to some embodiments. Four saturation bands, that is, S1 612, S2 616, S3 614, and S4 618, may be set around the rectangular ROI 130. The MRI device 1 requires four RF modules corresponding to the saturation bands and may sequentially generate sequences corresponding to the saturation bands.

Reference numeral 620 of FIG. 6 shows a multi-band set by the MRI device 1, according to some embodiments.

In 620 of FIG. 6, S1 612 and S3 624 may be saturation bands parallel to each other. In addition, S2 616 and S4 628 may be saturation bands parallel to each other. Therefore, the MRI device 1 may simultaneously generate saturation pulses corresponding to S1 612 and S3 624. Furthermore, saturation pulses corresponding to S2 616 and S4 628 may be simultaneously generated.

Furthermore, reference numeral 630 of FIG. 6 shows that the MRI device 1 differently applies the areas of saturation bands, according to some embodiments.

Four saturation bands, that is, S1 612, S2 616, S3 634, and S4 638, may be set around the rectangular ROI 130. Furthermore, the areas of S1 612 and S3 634 parallel to each other may be different from each other.

Reference numeral 640 of FIG. 6 shows that the MRI device 1 sets the multiband even when the areas of saturation bands parallel to each other are different from each other. Even when the areas of S1 612 and S3 644 parallel to each other are different from each other, the MRI device 1 may simultaneously generate saturation pulses corresponding to S1 612 and S3 644.

Reference numeral 710 of FIG. 7 shows a method, performed by the MRI device 1, of determining a saturation region based on coil geometry, according to some embodiments. The coil geometry may include at least one of the shape, position, or sensitivity of a receiving coil.

For example, when a user wants to image one leg 712 between both legs 712 and 714 of an object, the user may attach a leg receiving coil to the leg 712 to be imaged. At this time, the leg receiving coil may receive an MR signal generated from the other leg 714. In this case, even when the user sets an ROI 130 in a region 711 in which the sensitivity of the leg receiving coil is equal to or higher than a reference, the MR signal generated from the other leg 714 may intrude into the ROI 130. Therefore, artifacts may occur in an image of the leg 712.

When the MRI device 1 recognizes that the leg receiving coil is attached to only one leg 712, the MRI device 1 may prevent artifacts by automatically setting a saturation region 719 in a region corresponding to the other leg.

Reference numerals 720 and 730 of FIG. 7 show a method, performed by the MRI device 1, of changing a saturation region based on a user input of adjusting the number of saturation bands, according to some embodiments.

Referring to 720 of FIG. 7, when the MRI device 1 receives a user input of setting a hexagonal ROI, the MRI device 1 may set six saturation bands to be attached to six sides of the ROI so that no empty space is formed around the ROI. In this case, the saturation bands parallel to each other may be set as a multi-band pair.

Referring to 730 of FIG. 7, since a scan time increases as the number of saturation bands increases, a user may receive a user input of adjusting the number of saturation bands to four. When the MRI device 1 receives a user input of adjusting the number of saturation bands to four, the MRI device 1 may adjust the positions of the saturation bands so that an empty space between the four saturation bands and the ROI is minimized.

Reference numeral 740 of FIG. 7 shows a method, performed by the MRI device 1, of determining a saturation band based on an ROI, a signal region, and multi-band availability, according to some embodiments.

Referring to 740 of FIG. 7, the MRI device 1 may receive a user input of setting a first ROI 712 and a second ROI 714 on two legs 712 and 714 of an object, respectively. The MRI device 1 may determine the saturation band based on the ROI, the signal region, and the multi-band availability.

For example, the MRI device 1 may set two pairs of multi-bands 744, 745, 746, and 747 based on the positions of the first ROI 712, the second ROI 714, and the signal region. In this case, the MRI device 1 may set the multi-band so that the saturation region includes undesired signal regions 741, 742, and 743 around the ROls.

Reference numeral 750 of FIG. 7 shows a method, performed by the MRI device 1, of determining a saturation band based on the number of ROIs, according to some embodiments.

Referring to 750 of FIG. 7, when a plurality of ROIs are separated from each other, the MRI device 1 may determine the number and positions of saturation bands so that a space between the separated ROls and the saturation region becomes minimum and the multi-band is allowed to be set. In the case of 750 of FIG. 7, the saturation region may be determined so that three vertical saturation bands parallel to each other and two horizontal saturation bands parallel to each other surround the ROI.

FIG. 8 illustrates a method, performed by the MRI device 1, of providing guide information to allow an MR signal to be generated only from an ROI among pieces of guide information for preventing artifacts, according to some embodiments.

Referring to FIG. 8, the MRI device 1 may recommend an IVI method of generating an MR signal only from an ROI 130 of a signal region 120.

For example, when a user selects a spin echo sequence as an imaging sequence, the MRI device 1 may obtain data for one line of k-space by transmitting a 90-degree excite RF pulse to an object, transmitting a 180-degree refocus RF pulse to the object, and then receiving an MR signal received from the object.

In this case, only a region that has experienced both the 90-degree excite RF pulse and the 180-degree refocus RF pulse in the region of the object may emit an MR signal corresponding to the spin echo sequence, and only the MR signal corresponding to the spin echo sequence may be received by the MRI device 1.

In the case of the IVI method, the MRI device 1 may transmit RF pulses only to the ROI in a superimposed manner so that the MR signal corresponding to the applied sequence is allowed to be generated only from the ROI. For example, referring to FIG. 8, the MRI device 1 may transmit the 90-degree excite RF pulse and the 180-degree refocus RF pulse to the object so that the 90-degree excite RF pulse and the 180-degree refocus RF pulse superimpose in the ROI and do not superimpose in the other regions.

Therefore, since no MR signal is generated from the region other than the ROI, or an MR signal that is not the MR signal corresponding to the applied sequence is generated, the MRI device 1 may generate an MR image without aliasing.

The MRI device 1 may determine whether the IVI method is applicable, based on the imaging sequence and the shape of the ROI.

For example, in a gradient echo sequence, only one RF pulse is transmitted to the object so as to obtain data for one line of k-space. Therefore, RF pulses may not be set to superimpose only in the ROI, like the spin echo sequence. Therefore, the MRI device 1 may determine whether to recommend the IVI method considering the imaging sequence set by the user.

Furthermore, when the shape of the ROI is not rectangular, it is difficult to superimpose the RF pulses in the ROI. Therefore, the MRI device 1 may determine whether to recommend the IVI method considering the shape of the ROI.

When the MRI device 1 determines to recommend the IVI method, the MRI device 1 may display that the IVI method for preventing artifacts is applicable by displaying a region 810 of the 90-degree excite RF pulse and a region 820 of the 180-degree refocus RF pulse on a first image 110.

In addition, the MRI device 1 may display a button 830 for performing MR imaging based on the recommended IVI method.

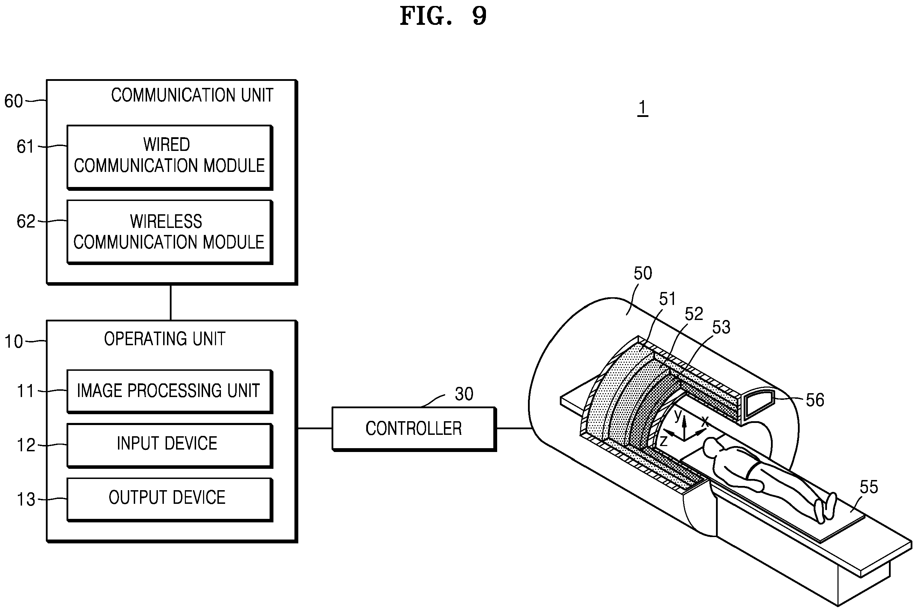

FIG. 9 is a schematic diagram of an MRI device 1.

Referring to FIG. 9, the MRI device 1 may include an operating unit 10, a controller 30, and a scanner 50. The controller 30 may be independently separated from the operating unit 10 and the scanner 50. Furthermore, the controller 30 may be separated into a plurality of sub-components and incorporated into the operating unit 10 and the scanner 50 in the MRI device 1. Operations of the components in the MRI device 1 will now be described in detail.

The scanner 50 may be formed to have a cylindrical shape (e.g., a shape of a bore) having an empty inner space into which an object may be inserted. A static magnetic field and a gradient magnetic field are created in the inner space of the scanner 50, and an RF signal is emitted toward the inner space.

The scanner 50 may include a static magnetic field generator 51, a gradient magnetic field generator 52, an RF coil unit 53, a table 55, and a display 56. The static magnetic field generator 51 creates a static magnetic field for aligning magnetic dipole moments of atomic nuclei of the object in a direction of the static magnetic field. The static magnetic field generator 51 may be formed as a permanent magnet or superconducting magnet using a cooling coil.

The gradient magnetic field generator 52 is connected to the controller 30 and generates a gradient magnetic field by applying a gradient to a static magnetic field in response to a control signal received from the controller 30. The gradient magnetic field generator 52 includes X, Y, and Z coils for generating gradient magnetic fields in X-, Y-, and Z-axis directions crossing each other at right angles and generates a gradient signal according to a position of a region being imaged so as to differently induce resonance frequencies according to regions of the object.

The RF coil unit 53 connected to the controller 30 may emit an RF signal toward the object in response to a control signal received from the controller 30 and receive an MR signal emitted from the object. In detail, the RF coil unit 53 may transmit, toward atomic nuclei of the object having precessional motion, an RF signal having the same frequency as that of the precessional motion, stop transmitting the RF signal, and then receive an MR signal emitted from the object.

The RF coil unit 53 may be formed as a transmitting RF coil for generating an electromagnetic wave having an RF corresponding to the type of an atomic nucleus, a receiving RF coil for receiving an electromagnetic wave emitted from an atomic nucleus, or one transmitting/receiving RF coil serving both functions of the transmitting RF coil and receiving RF coil. Furthermore, in addition to the RF coil unit 53, a separate coil may be attached to the object. Examples of the separate coil may include a head coil, a spine coil, a torso coil, and a knee coil according to a region being imaged or to which the separate coil is attached.

The display 56 may be disposed outside and/or inside the scanner 50. The display 56 is also controlled by the controller 30 to provide a user or the object with information related to medical imaging.

Furthermore, the scanner 50 may include an object monitoring information acquisition unit (not shown) configured to acquire and transmit monitoring information about a state of the object. For example, the object monitoring information acquisition unit may acquire monitoring information related to the object from a camera (not shown) for capturing images of a movement or position of the object, a respiration measurer (not shown) for measuring the respiration of the object, an ECG measurer for measuring the electrical activity of the heart, or a temperature measurer for measuring a temperature of the object and transmit the acquired monitoring information to the controller 30. The controller 30 may in turn control an operation of the scanner 50 based on the monitoring information. Operations of the controller 30 will now be described in more detail.

The controller 30 may control overall operations of the scanner 50.

The controller 30 may control a sequence of signals formed in the scanner 50. The controller 30 may control the gradient magnetic field generator 52 and the RF coil unit 53 according to a pulse sequence received from the operating unit 10 or a designed pulse sequence.

A pulse sequence may include all pieces of information required to control the gradient magnetic field generator 52 and the RF coil unit 53. For example, the pulse sequence may include information about a strength, a duration, and application timing of a pulse signal applied to the gradient magnetic field generator 52.

The controller 30 may control a waveform generator (not shown) for generating a gradient wave, i.e., an electrical pulse according to a pulse sequence and a gradient amplifier (not shown) for amplifying the generated electrical pulse and transmitting the same to the gradient magnetic field generator 52. Thus, the controller 30 may control formation of a gradient magnetic field by the gradient magnetic field generator 52.

Furthermore, the controller 30 may control an operation of the RF coil unit 53. For example, the controller 30 may supply an RF pulse having a resonance frequency to the RF coil unit 30 that emits an RF signal toward the object, and receive an MR signal received by the RF control unit 53. In this case, the controller 30 may adjust emission of an RF signal and reception of an MR signal according to an operating mode by controlling an operation of a switch (e.g., a T/R switch) for adjusting transmitting and receiving directions of the RF signal and the MR signal based on a control signal.

The controller 30 may control a movement of the table 55 where the object is placed. Before MRI is performed, the controller 30 may move the table 55 according to which region of the object is to be imaged.

The controller 30 may also control the display 56. For example, the controller 30 control the on/off state of the display 56 or a screen to be output on the display 56 according to a control signal.

The controller 30 may be formed as an algorithm for controlling operations of the components in the MRI device 1, a memory (not shown) for storing data in the form of a program, and a processor for performing the above-described operations by using the data stored in the memory. In this case, the memory and the processor may be implemented as separate chips. Alternatively, the memory and processor may be incorporated into a single chip.

The operating unit 10 may control overall operations of the MRI device 1 and include an image processing unit 11, an input device 12, and an output device 13.

The image processing unit 11 may control the memory to store an MR signal received from the controller 30, and generate image data with respect to the object from the stored MR signal by applying an image reconstruction technique by using an image processor.

For example, if a k space (for example, also referred to as a Fourier space or a frequency space) of the memory is filled with digital data to complete k-space data, the image processing unit 11 may reconstruct image data from the k-space data by applying various image reconstruction techniques (e.g., by performing inverse Fourier transform on the k-space data) by using the image processor.

Furthermore, the image processing unit 11 may perform various signal processing operations on MR signals in parallel. For example, image processing unit 11 may perform signal processing on a plurality of MR signals received via a multi-channel RF coil in parallel so as to convert the plurality MR signals into image data. In addition, the image processing unit 11 may store not only the image data in the memory, or the controller 30 may store the same in an external server via a communication unit 60 as will be described below.

The input device 12 may receive, from the user, a control command for controlling the overall operations of the MRI device 1. For example, the input device 12 may receive, from the user, object information, parameter information, a scan condition, and information about a pulse sequence. The input device 12 may be a keyboard, a mouse, a track ball, a voice recognizer, a gesture recognizer, a touch screen, or any other input device.

The output device 13 may output image data generated by the image processing unit 11. The output device 13 may also output a user interface (UI) configured so that the user may input a control command related to the MRI device 1. The output device 13 may be formed as a speaker, a printer, a display, or any other output device.

The output device 13 may be referred to as a display 13 and may display a first image of the object.

The controller 30 may determine a signal region of the object based on the first image of the object.

The input device 12 may be referred to as a user input device 12 and may receive user input of setting an ROI on the first image.

When the set ROI is part of the signal region of the object, the controller 30 may determine whether aliasing artifacts will occur in a second image to be generated based on the set ROI.

Furthermore, the controller 30 may determine a method of preventing artifacts based on coil geometry of the receiving coil, the shape of the ROI, anatomical information about the ROI, and the pulse sequence.

The controller 30 may control the display 13 to display the method of preventing artifacts.

The controller 30 may determine a predicted image based on the signal region and the ROI. Furthermore, the display 13 may display the predicted image.

The controller 30 may determine a position of a region where aliasing occurs in the signal region of the first image. Furthermore, the display 13 may display the position of the region where aliasing occurs in the signal region of the first image.

The controller 30 may control the display 13 to display information about a method of increasing the length of the ROI in the phase direction so that the ROI includes the signal region in the phase direction.

The controller 30 may control the display 13 to display information about a method of suppressing an MR signal emitted from a region other than the ROI in the signal region.

The controller 30 may control the display 13 to display information about a method of emitting an MR signal corresponding to an imaging sequence of a second image only from the ROI of the signal region.

The controller 30 may determine a pair of regions parallel to each other in the region surrounding the ROI and set a multi-saturation band for simultaneously suppressing MR signals emitted from the determined pair of regions.

The guide information includes a plurality of icons corresponding to a plurality of methods of preventing aliasing artifacts from occurring. When a user input of selecting one of the icons is received, the controller 30 may control the display 13 to display the guide information about the method corresponding to the selected icon.

The controller 30 may control the display 13 to display an estimated imaging time of each of the methods together with the icons corresponding to the methods.

Furthermore, although FIG. 9 shows that the operating unit 10 and the controller 30 are separate components, the operating unit 10 and the controller 30 may be included in a single device as described above. Furthermore, processes respectively performed by the operating unit 10 and the controller 30 may be performed by another component. For example, the image processing unit 11 may convert an MR signal received from the controller 30 into a digital signal, or the controller 30 may directly perform the conversion of the MR signal into the digital signal.

The MRI device 1 may further include a communication unit 60 and be connected to an external device (not shown) such as a server, a medical apparatus, and a portable device (e.g., a smartphone, a tablet PC, a wearable device, etc.) via the communication unit 60.

The communication unit 60 may include at least one component that enables communication with an external device. For example, the communication unit 60 may include at least one of a local area communication module (not shown), a wired communication module 61, and a wireless communication module 62.

The communication unit 60 may receive a control signal and data from an external device and transmit the received control signal to the controller 30 so that the controller 30 may control the MRI device 1 according to the received signal.

Alternatively, by transmitting a control signal to an external device via the communication unit 60, the controller 30 may control the external device according to the control signal.

For example, the external device may process data of the external device according to a control signal received from the controller 30 via the communication unit 60.

A program for controlling the MRI device 1 may be installed on the external device and may include instructions for performing some or all of the operations of the controller 30.

The program may be preinstalled on the external device, or a user of the external device may download the program from a server providing an application for installation. The server providing an application may include a recording medium having the program recorded thereon.

Embodiments may be implemented through non-transitory computer-readable recording media having recorded thereon computer-executable instructions and data. The instructions may be stored in the form of program codes, and when executed by a processor, generate a predetermined program module to perform a specific operation. Furthermore, when being executed by the processor, the instructions may perform specific operations according to the embodiments.

While one or more embodiments have been described with reference to the figures, it will be understood by those of ordinary skill in the art that various changes in form and details may be made therein without departing from the scope of the present disclosure as defined by the following claims. Accordingly, the above embodiments and all aspects thereof are examples only and are not limiting.

## Claims

1. A magnetic resonance imaging (MRI) device (1) comprising:
a display configured to display a first image of an object;
a controller configured to determine a signal region (120) of the object based on the first image of the object; and
a user input device configured to receive a user input of setting a region of interest (ROI) (130) on the first image,
wherein, when the set ROI is part of the signal region of the object, the controller is further configured to control the display to display guide information for preventing aliasing artifacts from occurring in a second image to be generated based on the set ROI
**characterised in that** the guide information includes a plurality of icons (162,164,166) corresponding to a plurality of methods of preventing the aliasing artifacts from occurring, and
when a user input of selecting one of the plurality of icons is received, the controller is further configured to display guide information about a method corresponding to the selected icon.

2. The MRI device of claim 1, wherein the controller is further configured to determine a predicted image based on the signal region and the ROI, and
the display is further configured to display the predicted image.

3. The MRI device of claim 1, wherein the display is further configured to display a position of a region where aliasing occurs in the signal region of the first image.

4. The MRI device of claim 1, wherein the controller is further configured to display the guide information by displaying information about a method of increasing a length of the ROI in a phase direction so that the ROI includes a signal region in the phase direction.

5. The MRI device of claim 1, wherein the controller is further configured to display the guide information by displaying information about a method of suppressing a magnetic resonance (MR) signal emitted from a region other than the ROI in the signal region.

6. The MRI device of claim 1, wherein the controller is further configured to display the guide information by displaying information about a method of emitting an MR signal corresponding to an imaging sequence of the second image from the ROI of the signal region.

7. The MRI device of claim 1, wherein
the controller is further configured to determine the guide information based on coil geometry of a receiving coil, a shape of the ROI, anatomical information about the ROI, and a pulse sequence.

8. A method, performed by an MRI device (1), of providing guide information, the method comprising
displaying a first image of an object;
determining a signal region (120) of the object based on the first image of the object
receiving a user input of setting a region of interest (ROI) (130) on the first image; and
when the set ROI is part of the signal region of the object, displaying guide information for preventing aliasing artifacts from occurring in a second image to be generated based on the set ROI, **characterised in that**
the guide information includes a plurality of icons (162,164,166) corresponding to a plurality of methods of preventing the aliasing artifacts from occurring, and
the displaying of the guide information for preventing the aliasing artifacts from occurring in the second image comprises,
when a user input of selecting one of the plurality of icons is received, displaying guide information about a method corresponding to the selected icon.

9. The method of claim 8, further comprising:
determining a predicted image based on the signal region and the ROI; and
displaying the predicted image.

10. The method of claim 8, further comprising
displaying a position of a region where aliasing occurs in the signal region of the first image.

11. The method of claim 8, wherein
the displaying of the guide information for preventing the aliasing artifacts from occurring in the second image comprises
displaying information about a method of increasing a length of the ROI in a phase direction so that the ROI includes a signal region in the phase direction.

12. The method of claim 8, wherein
the displaying of the guide information for preventing the aliasing artifacts from occurring in the second image comprises
displaying information about a method of suppressing an MR signal emitted from a region other than the ROI in the signal region.

13. The method of claim 8, wherein
the displaying of the guide information for preventing the aliasing artifacts from occurring in the second image comprises
displaying information about a method of emitting an MR signal corresponding to an imaging sequence of the second image from the ROI of the signal region.

## Patentansprüche

1. Magnetresonanztomographie (MRI)-Vorrichtung (1), umfassend:
ein Display, das so konfiguriert ist, dass es ein erstes Bild eines Objekts anzeigt;
eine Steuerung, die so konfiguriert ist, dass sie einen Signalbereich (120) des Objekts auf der Grundlage des ersten Bildes des Objekts bestimmt; und
eine Benutzereingabevorrichtung, die so konfiguriert ist, dass sie eine Benutzereingabe zum Einstellen eines Interessenbereichs (ROI) (130) auf dem ersten Bild empfängt,
wobei, wenn die eingestellte ROI ein Teil des Signalbereichs des Objekts ist, die Steuereinheit ferner so konfiguriert ist, dass sie die Anzeige steuert, um Führungsinformationen anzuzeigen, um zu verhindern, dass Aliasing-Artefakte in einem zweiten Bild auftreten, das auf der Grundlage der eingestellten ROI erzeugt werden soll.
**dadurch gekennzeichnet, dass**
die Führungsinformation eine Vielzahl von Symbolen (162, 164, 166) enthält, die einer Vielzahl von Verfahren zur Verhinderung des Auftretens von Aliasing-Artefakten entsprechen, und
wenn eine Benutzereingabe zur Auswahl eines der Vielzahl von Symbolen empfangen wird, ist die Steuereinheit ferner so konfiguriert, dass sie Leitinformationen über ein Verfahren anzeigt, das dem ausgewählten Symbol entspricht.

2. MRI-Gerät nach Anspruch 1, wobei die Steuereinheit ferner so konfiguriert ist, dass sie ein vorhergesagtes Bild auf der Grundlage des Signalbereichs und der ROI bestimmt, und
die Anzeige ist außerdem so konfiguriert, dass sie das vorhergesagte Bild anzeigt.

3. MRI-Gerät nach Anspruch 1, wobei die Anzeige ferner so konfiguriert ist, dass sie eine Position eines Bereichs anzeigt, in dem Aliasing im Signalbereich des ersten Bildes auftritt.

4. MRI-Gerät nach Anspruch 1, wobei die Steuerung ferner so konfiguriert ist, dass sie die Führungsinformationen anzeigt, indem sie Informationen über ein Verfahren zur Vergrößerung einer Länge der ROI in einer Phasenrichtung anzeigt, so dass die ROI einen Signalbereich in der Phasenrichtung umfasst.

5. MRI-Gerät nach Anspruch 1, wobei die Steuereinheit ferner so konfiguriert ist, dass sie die Führungsinformationen anzeigt, indem sie Informationen über ein Verfahren zur Unterdrückung eines Magnetresonanzsignals (MR-Signals) anzeigt, das von einem anderen Bereich als der ROI im Signalbereich ausgesendet wird.

6. MRI-Gerät nach Anspruch 1, wobei die Steuereinheit ferner so konfiguriert ist, dass sie die Führungsinformationen anzeigt, indem sie Informationen über ein Verfahren zum Aussenden eines MR-Signals anzeigt, das einer Bildgebungssequenz des zweiten Bildes von der ROI des Signalbereichs entspricht.

7. MRI-Gerät nach Anspruch 1, wobei
der Controller ist ferner so konfiguriert, dass er die Führungsinformationen auf der Grundlage der Spulengeometrie einer Empfangsspule, einer Form des ROI, anatomischer Informationen über den ROI und einer Impulsfolge bestimmt.

8. Verfahren, das von einer MRI-Vorrichtung (1) durchgeführt wird, um Führungsinformationen zu liefern, wobei das Verfahren umfasst:
die Anzeige eines ersten Bildes eines Objekts;
Bestimmung eines Signalbereichs (120) des Objekts auf der Grundlage des ersten Bildes des Objekts;
Empfangen einer Benutzereingabe zum Festlegen einer Region von Interesse (ROI) (130) auf dem ersten Bild; und
wenn die eingestellte ROI Teil des Signalbereichs des Objekts ist, Anzeigen von Führungsinformationen zum Verhindern des Auftretens von Aliasing-Artefakten in einem zweiten Bild, das auf der Grundlage der eingestellten ROI erzeugt werden soll, **dadurch gekennzeichnet, dass**
die Führungsinformation eine Vielzahl von Symbolen (162, 164, 166) enthält, die einer Vielzahl von Verfahren zur Verhinderung des Auftretens von Aliasing-Artefakten entsprechen, und
die Anzeige der Führungsinformation zur Verhinderung des Auftretens von Aliasing-Artefakten in dem zweiten Bild umfasst,
wenn eine Benutzereingabe zur Auswahl eines der mehreren Symbole empfangen wird, Anzeige von Leitinformationen über ein Verfahren, das dem ausgewählten Symbol entspricht.

9. Verfahren nach Anspruch 8 umfasst ferner:
Bestimmen eines vorhergesagten Bildes basierend auf dem Signalbereich und der ROI; und
und zeigt das vorhergesagte Bild an.

10. Verfahren nach Anspruch 8 umfasst ferner
Anzeige der Position eines Bereichs, in dem Aliasing im Signalbereich des ersten Bildes auftritt.

11. Verfahren nach Anspruch 8, wobei
die Anzeige der Führungsinformation zur Verhinderung des Auftretens von Aliasing-Artefakten in dem zweiten Bild umfasst
Anzeige von Informationen über ein Verfahren zur Vergrößerung der Länge der ROI in einer Phasenrichtung, so dass die ROI einen Signalbereich in der Phasenrichtung umfasst.

12. Verfahren nach Anspruch 8, wobei
die Anzeige der Führungsinformation zur Verhinderung des Auftretens von Aliasing-Artefakten in dem zweiten Bild umfasst
Anzeige von Informationen über ein Verfahren zur Unterdrückung eines MR-Signals, das von einem anderen Bereich als der ROI im Signalbereich ausgesendet wird.

13. Verfahren nach Anspruch 8, wobei
die Anzeige der Führungsinformation zur Verhinderung des Auftretens von Aliasing-Artefakten in dem zweiten Bild umfasst
Anzeigen von Informationen über ein Verfahren zum Aussenden eines MR-Signals, das einer Bildgebungssequenz des zweiten Bildes von der ROI des Signalbereichs entspricht.

## Revendications

1. Dispositif d'imagerie par résonance magnétique (IRM) (1) comprenant:
un écran configuré pour afficher une première image d'un objet;
un contrôleur configuré pour déterminer une région de signal (120) de l'objet sur la base de la première image de l'objet; et
un dispositif d'entrée utilisateur configuré pour recevoir une entrée utilisateur de définition d'une région d'intérêt (ROI) (130) sur la première image,
dans lequel, lorsque le ROI défini fait partie de la région de signal de l'objet, le contrôleur est en outre configuré pour commander l'affichage pour afficher des informations de guide pour empêcher des artefacts de crénelage de se produire dans une seconde image à générer sur la base du ROI défini.
**caractérisé en ce que**
les informations de guidage comprennent une pluralité d'icônes (162, 164, 166) correspondant à une pluralité de méthodes pour empêcher les artefacts de crénelage de se produire, et
lorsqu'une entrée utilisateur consistant à sélectionner l'une de la pluralité d'icônes est reçue, le contrôleur est en outre configuré pour afficher des informations de guide concernant un procédé correspondant à l'icône sélectionnée.

2. Dispositif IRM selon la revendication 1, dans lequel le contrôleur est en outre configuré pour déterminer une image prédite sur la base de la région de signal et du ROI, et
l'affichage est en outre configuré pour afficher l'image prédite.

3. Dispositif IRM selon la revendication 1, dans lequel l'affichage est en outre configuré pour afficher une position d'une région où le crénelage se produit dans la région de signal de la première image.

4. Dispositif d'IRM de la revendication 1, dans lequel le contrôleur est en outre configuré pour afficher les informations de guidage en affichant des informations concernant un procédé d'augmentation d'une longueur de la ROI dans une direction de phase de sorte que la ROI comprenne une région de signal dans la direction de phase .

5. Dispositif IRM selon la revendication 1, dans lequel le contrôleur est en outre configuré pour afficher les informations de guidage en affichant des informations concernant un procédé de suppression d'un signal de résonance magnétique (MR) émis par une région autre que le ROI dans la région de signal.

6. Dispositif IRM selon la revendication 1, dans lequel le contrôleur est en outre configuré pour afficher les informations de guidage en affichant des informations sur un procédé d'émission d'un signal RM correspondant à une séquence d'imagerie de la seconde image à partir du ROI de la région de signal.

7. Dispositif IRM de la revendication 1, dans lequel
le contrôleur est en outre configuré pour déterminer les informations de guidage sur la base de la géométrie de bobine d'une bobine de réception, d'une forme de la ROI, d'informations anatomiques sur la ROI et d'une séquence d'impulsions.

8. Procédé, exécuté par un dispositif IRM (1), de fourniture d'informations de guidage, le procédé comprenant:
l'affichage d'une première image d'un objet;
déterminer une région de signal (120) de l'objet sur la base de la première image de l'objet;
recevoir une entrée d'utilisateur pour définir une région d'intérêt (ROI) (130) sur la première image ; et
lorsque le ROI défini fait partie de la région de signal de l'objet, afficher des informations de guidage pour empêcher l'apparition d'artefacts de crénelage dans une deuxième image à générer sur la base du ROI défini, **caractérisé en ce que**
les informations de guidage comprennent une pluralité d'icônes (162, 164, 166) correspondant à une pluralité de méthodes pour empêcher les artefacts de crénelage de se produire, et
l'affichage de l'information de guidage pour empêcher les artefacts de crénelage de se produire dans la seconde image comprend,
lorsqu'une entrée utilisateur consistant à sélectionner l'une de la pluralité d'icônes est reçue, afficher des informations de guide concernant un procédé correspondant à l'icône sélectionnée.

9. Procédé de la revendication 8, comprenant en outre :
déterminer une image prédite basée sur la région de signal et le ROI ; et
affichant l'image prédite.

10. Procédé de la revendication 8, comprenant en outre
afficher une position d'une région où le crénelage se produit dans la région de signal de la première image.

11. Procédé de la revendication 8, dans lequel
l'affichage des informations de guidage pour empêcher les artefacts de crénelage de se produire dans la seconde image comprend
afficher des informations sur un procédé d'augmentation d'une longueur de la ROI dans une direction de phase de sorte que la ROI inclut une région de signal dans la direction de phase.

12. Procédé de la revendication 8, dans lequel
l'affichage des informations de guidage pour empêcher les artefacts de crénelage de se produire dans la seconde image comprend
afficher des informations sur un procédé de suppression d'un signal MR émis par une région autre que la ROI dans la région de signal.

13. Procédé de la revendication 8, dans lequel
l'affichage des informations de guidage pour empêcher les artefacts de crénelage de se produire dans la seconde image comprend
afficher des informations sur un procédé d'émission d'un signal MR correspondant à une séquence d'imagerie de la seconde image à partir du ROI de la région de signal.
